# EUROPÄISCHE PATENTANMELDUNG

(11) **EP 3 760 608 A1**
(43) Veröffentlichungstag der Anmeldung: **06.01.2021**
(21) Anmeldenummer: 19184618.7
(22) Anmeldetag: 05.07.2019
(51) Int. Cl.: C07C 67/39, C07C 69/54, C07C 67/08, C07C 51/25, C07C 57/04, C07C 45/35, C07C 45/37, C07C 47/22

(54) **VERFAHREN ZUR HERSTELLUNG VON ALKYLMETHACRYLATEN UND OPTIONAL METHACRYLSÄURE**

(71) Anmelder: Röhm GmbH, 64295 Darmstadt (DE)
(72) Erfinder: Die Erfindernennung liegt noch nicht vor
(74) Vertreter: Röhm Patent Association

(57) **Zusammenfassung**

Die vorliegende Erfindung betrifft ein neuartiges Verfahren zur Herstellung von Methacrylaten, wie Methacrylsäure und/oder Alkylmethacrylaten, insbesondere MMA. Insbesondere betrifft die vorliegende Erfindung eine Ausbeutensteigerung und Effizienzsteigerung von C-4 basierten Herstellungsverfahren, also insbesondere solchen Verfahren, die von Isobutylen oder tert-Butanol als Rohstoff ausgehen.

Dabei ist es mit dem vorliegenden erfindungsgemäßen Verfahren gelungen, solche Verfahren bei gleichbleibenden oder sogar gesteigerten Aktivitäten und Selektivitäten länger störungsfrei zu betreiben. Hieraus ergibt sich die Möglichkeit, solche Verfahren möglichst einfach, wirtschaftlich und umweltschonend durchzuführen.

## Beschreibung

### Gebiet der Erfindung

Die vorliegende Erfindung betrifft ein neuartiges Verfahren zur Herstellung von Methacrylaten, wie Methacrylsäure und/oder Alkylmethacrylaten, insbesondere MMA. Insbesondere betrifft die vorliegende Erfindung eine Ausbeutensteigerung und Effizienzsteigerung von C-4 basierten Herstellungsverfahren, also insbesondere solchen Verfahren, die von Isobutylen oder tert-Butanol als Rohstoff ausgehen.

Dabei ist es mit dem vorliegenden erfindungsgemäßen Verfahren gelungen, solche Verfahren bei gleichbleibenden oder sogar gesteigerten Aktivitäten und Selektivitäten länger störungsfrei zu betreiben. Hieraus ergibt sich die Möglichkeit, solche Verfahren möglichst einfach, wirtschaftlich und umweltschonend durchzuführen.

### Stand der Technik

Methylmethacrylat (MMA) wird heute beispielsweise ausgehend von Blausäure und Aceton über das entstehende Acetoncyanhydrin (ACH) als Zentralintermediat hergestellt. Dieses Verfahren hat den Nachteil, dass sehr große Mengen an Ammoniumsulfat erhalten werden, deren Aufbereitung mit sehr hohen Kosten verbunden ist. Weitere Verfahren, die eine andere Rohstoffbasis als ACH verwenden, sind in der einschlägigen Patentliteratur beschrieben und mittlerweile im Produktionsmaßstab realisiert worden. In diesem Zusammenhang werden heute auch C-4 basierte Rohstoffe wie Isobutylen oder tert-Butanol als Edukte verwendet, die über mehrere Verfahrensstufen in die gewünschten Methacrylsäurederivate umgewandelt werden.

Hierbei wird im Allgemeinen Isobutylen oder tert-Butanol zu Methacrolein in einer ersten Stufe oxidiert, welches anschließend zu Methacrylsäure mit Sauerstoff umgesetzt wird. Die erhaltene Methacrylsäure wird nachfolgend mit Methanol in MMA überführt. Nähere Einzelheiten zu diesem Verfahren sind unter anderem in Ullmann's Encyclopedia of Industrial Chemistry 2012, Wiley-VCH Verlag GmbH & Co. KGaA, Weinheim, Methacrylic Acid and Derivatives, DOI: 10.1002/14356007.a16_441.pub2 sowie in Krill und Rühling et. al. "Viele Wege führen zum Methacrylsäuremethylester", WILEY-VCH Verlag GmbH & Co. KGaA, Weinheim, doi.org/10.1002/ciuz.201900869 dargelegt.

Prinzipiell sind als Stand der Technik für vorliegende Erfindung auf C-4 Rohstoffen basierende MMA Verfahren relevant. Man unterscheidet drei Prozesse zur Herstellung von MMA auf dieser Basis. Als Rohstoffe werden z.B. tert-Butanol, das durch Wasser Eliminierung zu Isobuten umgewandelt wird, alternativ Methyl-tert-butylether, welches durch Methanol-Eliminierung zu Isobuten umgesetzt wird, oder Isobuten selbst, welches beispielsweise aus einem Cracker als Rohstoff zur Verfügung steht, eingesetzt. Zusammengefasst ergeben sich daraus die folgenden drei Routen:
Verfahren A, "Tandem C₄ Direct Oxidation" Verfahren, ohne Zwischenisolierung von Methacrolein): Hier wird in einem ersten Schritt aus Isobuten Methacrolein hergestellt, das in einem Schritt 2 zu Methacrylsäure oxidiert wird, bevor schließlich diese in einem Schritt 3 zu MMA oxidiert wird.
Verfahren B, "Separate C₄ Direct Oxidation" Verfahren: Hier wird soweit identisch in einem ersten Schritt aus Isobuten Methacrolein hergestellt, das in einem Schritt 2 zunächst isoliert und zwischengereinigt wird, bevor es in Schritt 3 zu Methacrylsäure und diese schließlich in einem Schritt 4 zu MMA oxidiert wird.
Verfahren C, "Direct Metha Process" oder Direct Oxidative Esterification Process: Auch hier wird in einem ersten Schritt aus Isobuten Methacrolein hergestellt, welches auch hier in einem Schritt 2 zunächst isoliert und zwischengereinigt wird, bevor es in einem Schritt 3 direkt oxidativ zu MMA verestert wird.

Alle dargestellten Verfahren sind im Stand der Technik gut dokumentiert, unter anderem in (i) IHS Chemical Process Economics Program, Review 2015-05, R.J. Chang, Syed Naqvi (ii) Vapor Phase Catalytic Oxidation of Isobutene to Methacrylic Acid, Stud. Surf. Sci. Catal. 1981, 7, 755-767 (iii)

### Zum "Tandem Verfahren", Verfahren A:

Zunächst wird Isobuten mit Luft, optional recycliertem gasförmigen Strömen und Wasserdampf in der Gasphase bei Temperaturen zwischen 300 und 400°C in einem Rohrbündelreaktor an einem Sohio ähnlichen Kontakt auf bi-Molybdän Basis zu Methacrolein umgesetzt. Das entstehende Prozessgas wird zwischengekühlt, nochmal mit Luft und Wasserdampf versetzt und mit recycliertem Methacrolein aus der zweiten Stufe versetzt. Dieses so eingestellte Gasgemisch wird direkt auf einen zweiten Rohbündelreaktor mit einem zweiten Kontakt, bei dem es sich um eine modifizierte Heteropolysäure mit Dotierungen durch andere Metalloxide handelt, bei Temperaturen von 260 bis 360°C zu Methacrylsäure umgesetzt. Charakteristisch an diesem Verfahren ist, dass die erste Reaktion bei fast quantitativen Umsätzen - bezogen auf Isobuten meint dies Umsätze zwischen 98 und 99,9% - gefahren werden muss. Dies ist nötig, da das Isobuten die Aktivität der nachfolgenden Oxidation hemmt. Weiterhin wird in der zweiten Stufe der Umsatz des Methacrolein auf 60 bis 90% eingeschränkt, so dass im Prozessgas der zweiten Stufe immer noch substanzielle Mengen an nicht umgesetztem Methacrolein vorhanden sind. Das Prozessgas dieser zweiten Stufe wird gequencht und entstandene Methacrylsäure von nicht umgesetztem Methacrolein getrennt. Das nicht umgesetzte Methacrolein wird kondensiert und in einer zwar konzentrierten, aber nicht reinen Form wiederum verdampft und zwischen die beiden Gasphasenreaktoren wieder eingespeist. Namensgebend für den Tandem Prozess ist also die Tatsache, dass das Methacrolein aus dem Prozessgas der ersten Stufe nicht aufgearbeitet wird, wohl aber das recyclierte Methacrolein aus der zweiten Oxidationsstufe.

### Zum "Separate C4 Direct Oxidation Verfahren", Verfahren B:

Zunächst wird Isobuten mit Luft, optional recycliertem gasförmigen Strömen und Wasserdampf in der Gasphase bei Temperaturen zwischen 300 und 400°C in einem Rohrbündelreaktor an einem Sohio ähnlichen Kontakt auf bi-Molybdän Basis zu Methacrolein umgesetzt. In diesem Verfahren wird jetzt Methacrolein nach dem Austrag aus Reaktor 1 gekühlt und kondensiert. Zusätzlich wird die als Nebenprodukt gebildete Methacrylsäure abgetrennt und das Methacrolein destillativ isoliert. Nach dieser mehrstufigen Operation erhält man flüssiges Methacrolein das nun erneut verdampft werden muss und mit Wasserdampf und Luft gegebenenfalls Recyclegas für die zweite Oxidationsstufe konditioniert wird. An dieser Stelle wird jetzt ebenfalls recycliertes Methacrolein mit eingespeist. Dieses so eingestellte Gasgemisch wird auf einen zweiten Rohbündelreaktor mit einem zweiten Kontakt, bei dem es sich um eine modifizierte Heteropolysäure mit Dotierungen aus anderen Metalloxiden handelt, bei Temperaturen zwischen 260 und 360°C zu Methacrylsäure umgesetzt. Charakteristisch an diesem Verfahren ist somit, dass die erste Reaktion nicht mehr bei quantitativen Umsätzen, sondern bei einem Isobuten-Umsatz von mindestens 90 bis 98% gefahren werden kann. Auch vorteilhaft ist, dass die Reaktion bis zu einem Umsatz mit der maximal günstigen Kombination aus Selektivität und Ausbeute bezüglich MAL aus Isobuten durchgeführt werden kann. Weiterhin wird auch hier in der zweiten Stufe der Umsatz des Methacrolein auf 60 bis 90% eingeschränkt, so dass im Prozessgas der zweiten Stufe immer noch substanzielle Mengen an nicht umgesetztem Methacrolein vorhanden sind. Das Prozessgas dieser zweiten Stufe wird gequencht und entstandene Methacrylsäure von nicht umgesetztem Methacrolein getrennt. Das nicht umgesetzte Methacrolein wird kondensiert und in einer zwar konzentrierten, aber nicht reinen Form wiederum verdampft und vor den zweiten Gasphasenreaktor wieder eingespeist. Namensgebend für den "Separate C4 Direct Oxidation" Prozess ist die Tatsache, dass das Methacrolein, sowie das recycelte Methacrolein aus der zweiten Oxidationsstufe aus dem Prozessgas der ersten Stufe aufgearbeitet und zwischenisoliert werden.

Verfahren A und B ist gemeinsam, dass Methacrylsäure das Hauptprodukt ist. Optional kann die bei beiden Verfahren mehr oder weniger reine Methacrylsäure in einer Veresterungsreaktion mit Methanol zu Methylmethacrylat umgesetzt werden. Üblicherweise verwendet man für diesen letzten Schritt einen Brönstedt aktiven Katalysator, das kann in der homogenen Variante eine gelöste starke Säure sein, wie beispielsweise Schwefelsäure oder Methansulfonsäure, oder ein sauerer lonentauscher, der entsprechende heterogene Säurefunktionen aufweist.

Verfahren C, der "Direct Metha Process", verläuft in den ersten beiden Schritten analog dem Verfahren B. Nach Gasphasenoxidation von Isobuten oder tert-Butylalkohol wird Roh-Methacrolein flüssig aufgearbeitet. Darauf folgt ein für diese Variante charakteristischer, sich deutlich unterscheidender Schritt der sogenannten direkten oxidativen Veresterung von Methacrolein mit Methanol und sauerstoffhaltigem Gas, wie z.B. Luft, an einem heterogenen pulverartigen Edelmetall-Kontakt in flüssiger Phase. Das Verfahren gelangt direkt zu MMA und durchläuft nicht den Zwischenschritt der Methacrylsäure-Herstellung in der Gasphase, wie in Verfahren A oder B.

Dieses von ASAHI entwickelte Verfahren ist unter anderem in den Druckschriften US 5,969,178 und US 7,012,039 beschrieben. Nachteil dieses Verfahrens ist insbesondere ein sehr hoher Energiebedarf.

In US 5,969,178 ist ein Verfahren zur oxidativen Umsetzung von Isobuten oder tert-Butanol zu Methacrolein und die folgende oxidative Veresterung zu MMA beschrieben. In dieser zweiten Stufe wird eine flüssige, im Wassergehalt reduzierte Mischung aus Methacrolein und Methanol mit molekularem Sauerstoff und einem Palladiumkatalysator umgesetzt, wobei dieser zumeist auf einem Träger als Palladium- Blei-Katalysator vorliegt. Gemäß US 6,040,472 führt ein solcher Pd-Pb-Katalysator bei einem optimalen Palladium-Anteil von 5 % zu einer MMA-Selektivität von bis 91 % und zu einer Raum-Zeit-Ausbeute von 5,3 mol MMA / h*kg Katalysator. Palladium(-Blei) Katalysatoren haben jedoch den Nachteil, dass es in einem kontinuierlichen Betrieb zu hohen Verlusten der Bleikomponente kommt (so genanntes Leaching). Auf der einen Seite, führt dies zu einer aufwendigen Abwasserentsorgung, auf der anderen Seite müssen Bleisalze dem System kontinuierlich zugeführt werden.

EP 2 177 267 und EP 2 210 664 beschreiben Nickeloxid-Katalysatoren mit einem Goldanteil zwischen 1 und 80 mol%, die auf einem Trägermaterial vorgelegt werden, für die oxidative Veresterung von Aldehyden zu Estern. Diese Katalysatoren werden mit einem Durchmesser zwischen 10 und 200 µm eingesetzt. Insbesondere liegen diese Partikel mit einer Schalenstruktur vor, bei der das Nickeloxid sich auf der Oberfläche und das Gold auf einer innenliegenden Schicht befindet. Diese Katalysatoren führen bestenfalls zu einer MMA-Selektivität von bis 97,1 % bei einer Raum-Zeit-Ausbeute von 9,6 mol MMA / h*kg Katalysator.

EP 2 210 664 offenbart dazu eine spezielle Variante, bei der Katalysatorpartikel im Nanometerbereich auf einen Supportpartikel mit einem Durchmesser zwischen 10 und 200 µm aufgebracht sind. In einer Variante hat dieser Supportpartikel eine Größe von 3 mm. Der Katalysator kann auch zylinderförmig oder in Wabenform in einem Festbettreaktor vorgelegt werden. Weiter wird die Prozessführung in einer solchen Reaktorvariante nicht beschrieben.

In EP 1 393 800 werden Goldpartikel oder goldhaltige Partikel mit einem Durchmesser von kleiner 6 nm auf einem Trägermaterial, insbesondere auf einem Metalloxid, als Katalysator beschrieben. Selektivitäten zu MMA von bis zu 93 % und Raumzeitausbeuten von bis zu 50,7 mol MMA / h*kg Katalysator bei einem Goldgehalt der Katalysatorpartikel von 4,5 mol% erhalten. Darüber hinaus ist der Offenbarungsgehalt dem der EP 2 210 664 analog.

Als Nebenprodukt der MAL-Synthese bildet sich Methacrylsäure und der pH-Wert des Reaktionsgemisches sinkt entsprechend. Dies führt zu weiteren Problemen. So wird mit sinkendem pH-Wert in steigenden Mengen das Nebenprodukt 1,1-Dimethoxyisobuten (DMI) als Acetal aus Methacrolein und Methanol gebildet. Damit steht ein Teil des Methacroleins in Form eines Dimethylacetals für die weitere Umsetzung zum MMA nicht mehr zur Verfügung, und die Raumzeitausbeute der MMA-Synthese sinkt entsprechend. Das Dimethylacetal bereitet darüber hinaus in der darauf folgenden destillativen Aufarbeitung des MMA Probleme. Außerdem wirkt ein Gemisch mit einem zu geringen pH-Wert negativ auf die Stabilität und Lebensdauer des eingesetzten Katalysators (Leaching, Änderung der Porenstruktur des Katalysators usw.). So lehrt in Bezug auf die Untergrenze von pH = 5 die JP 2003048863, dass man eine basische Lösung zum Ausgleich des pH-Werts zugeben kann. Diese basische Lösung, z.B. in Form einer NaOH-Lösung, selbst weist in der Regel einen pH-Wert größer 10 auf.

Alle diese Verfahren betreffen Syntheserouten, die von C-4 Rohstoffen (Isobuten oder Tertbutylalkohol) ausgehen.

Zu diesen Varianten (Verfahren A, B und C) kann man zusammengefasst feststellen, dass nach Stand der Technik durch vielfache technische und kommerzielle Mängel sowie Defizite bestehen. So werden bei der zweifachen Gasphasenoxidation pro Prozessstufe etwa 75-85% Ausbeute erzielt. Somit wird nur eine Gesamtausbeute an Methacrylsäure von knapp 65% (+/-5 %) erreicht. Dies gilt insgesamt für alle Ausführungsformen, wie z.B. für so genannte Tandem-Verfahren, wie sie großtechnisch beispielsweise von Nippon Kayaku und Mitsui beschrieben werden, als auch für Ausführungsformen mit Zwischenisolierung von Methacrolein, wie von Mitsubishi Rayon beschrieben.

Zudem gibt es eine erhebliche technische Herausforderung bei diesen Verfahren was die sicherheitstechnische Ausführung betrifft. Bei der Verdampfung des sogenannten Recycle Methacroleins, also Methacrolein, welches in der zweiten Oxidationsstufe nicht umgesetzt wird, und dessen Injektion zwischen die beiden Oxidationsreaktoren, arbeitet man im so genannten "mageren" Bereich sehr nahe an der Explosionsgrenze des Gemischs. Wenn dieses kritische Gemisch mit dem Prozessgas der ersten Reaktorstufe z.B. bei Temperaturen von üblicherweise über 300°C aufgegeben wird, kommt es zu teerartigen Ablagerungen und problematischen Zusammensetzungen des Feedgases vor dem zweiten Oxidationsreaktor. Diese Problematik ist in einer Vielzahl von Patenten und Lösungsvorschlägen beschrieben, der nur durch komplexe und teure Maßnahmen gegengesteuert werden kann. Weiterhin ist die Problematik vor allem bei einem kontinuierlichen Betrieb nicht ausreichend auszugleichen.

Zusammengefasst kann man feststellen, dass bislang kein Verfahren basierend auf C4-Rohstoff beschrieben ist, bei dem der kritische Schritt der Abtrennung und Reaktion von nicht umgesetzten Methacrolein im Prozessgas der zweiten, zumeist mit einer Heteropolysäure katalysierten Umsetzung nicht dazu führt, dass dieses recyclierbare Methacrolein wieder in die zweite Heteropolysäure-katalysierte Oxidation eingespeist wird. Aus dem Stand der Technik sind somit nur Verfahren bekannt, die dieses recyclierbare Methacrolein zu Methacrylsäure umsetzen, mit allen beschriebenen Nachteilen und technischen Problemen bezüglich Ausbeute und der sicherheitstechnischen Umsetzung der Rückführung vor die zweite Stufe. Es war daher insbesondere wünschenswert, eine hocheffiziente Technologie zu entwickeln, die es erlaubt dieses Recycle Methacrolein zu Methylmethacrylat umzusetzen, ohne den Umweg über Methacrylsäure zu gehen.

Es besteht somit vor allem erheblicher Verbesserungsbedarf, was die oxidative Umsetzung des Recycle-Methacroleins in diesen C4-basierten Verfahren betrifft.

Allen diesen Verfahren basierend auf C4-Ausgangsmaterialien ist gemeinsam, dass die auf die MAL-Herstellung folgenden oxidativen Stufen zur Methacrylsäure relativ ineffizient sind und keine der Ausführungen eine Endausbeute oberhalb von 70% ermöglicht. Daher besteht ein hoher Bedarf an einer alternativen Verfahrensführung, die eine deutliche Effizienzsteigerung ermöglicht. Darüber hinaus führen einige der Nebenprodukte der C4-Verfahren zu Ablagerungen in der Anlage. Diese Ablagerungen können je nach Nebenprodukt direkt erfolgen oder aber durch eine Polymerisation eines relativ reaktiven Nebenproduktes erfolgen. Auch hier besteht der Bedarf die Gesamtmenge gebildeter Nebenprodukte, insbesondere derer, die problematische Ablagerungen bilden können, zu reduzieren.

### Aufgabe

Aufgabe der vorliegenden Erfindung war es, ein neues Verfahren zur Herstellung von Alkylmethacrylaten, insbesondere von MMA zur Verfügung zu stellen, welches von C4-Bausteinen ausgeht und eine besonders hohe Gesamtausbeute aufweist.

Eine weitere Aufgabe der vorliegenden Erfindung war es, einen insgesamt sehr hohen Gesamtumsatz der Edukte zu realisieren und dabei möglichst geringe Abfallmengen zu bilden.

Darüber hinaus war es Aufgabe der vorliegenden Erfindung, ein Alkylmethacrylat, insbesondere MMA herzustellen, dass nur geringe Konzentrationen an spezifischen Nebenprodukten aufweist.

Weiterhin war es die Aufgabe der vorliegenden Erfindung, ein Verfahren zu realisieren, mit dem kontinuierlich und großtechnisch sehr flexibel verschiedene Alkylmethacrylate und/oder Methacrylsäure, wenn gewünscht sogar gleichzeitig, produziert werden können.

Weitere nicht explizit genannte Aufgaben, können sich aus den Ansprüchen und der folgenden Beschreibung der Erfindung ergeben, ohne hier explizit aufgeführt zu sein.

### Beschreibung

Gelöst werden die Aufgaben durch ein neuartiges Verfahren zur Herstellung von Alkylmethacrylaten und optional Methacrylsäure, insbesondere von Methylmethacrylat (MMA) aus C-4-Rohstoffen, insbesondere in einem Verfahren mit Methacrolein als Intermediat.

Das erfindungsgemäße Verfahren basiert auf einem Verfahren, bei dem in einer ersten Reaktionsstufe in einem Reaktor 1 Methacrolein durch eine Partialoxidation in der Gasphase hergestellt wird und dieses in einer zweiten Reaktionsstufe in einem Reaktor 2 durch Partialoxidation in der Gasphase zu Methacrylsäure umgesetzt wird. Dabei zeichnet sich die erfindungsgemäße Entwicklung dadurch aus, dass das in der zweiten Reaktionsstufe im Reaktor 2 nicht umgesetzte Methacrolein von der entstehenden Methacrylsäure abgetrennt wird und in einem weiteren Oxidationsschritt in einem Reaktor 4 in der Flüssigphase in Anwesenheit von einem Alkohol oxidativ verestert wird.

Weiterhin wird die in Reaktor 2 entstandene rohe Methacrylsäure destillativ und/oder extraktiv aufgereinigt und/oder in einem weiteren Schritt mit einem Alkohol in einem Reaktor 3 sauer katalysiert zum Alkylmethacrylat umgesetzt.

Bevorzugt ist eine Variante des erfindungsgemäßen Verfahrens, bei der folgende Prozessschritte durchlaufen werden:
A) Herstellung von Methacrolein aus Isobuten und/oder tert-Butanol in Gegenwart von Wasserdampf und Sauerstoff in einem Reaktor 1, wobei die Reaktion in der Gasphase an einem heterogenen Kontakt I erfolgt, und wobei ein Methacrolein-haltiges Prozessgas 1 erhalten wird.
B) Überführung des Prozessgases 1 aus Verfahrensschritt A), enthaltend Methacrolein und Wasserdampf, aus Reaktor 1 unter Zuführung eines sauerstoffhaltigen Gases und optional weiteren Wasserdampfes in einen Reaktor 2, wobei eine Reaktion an einem heterogenen Kontakt II erfolgt, und ein Methacrylsäure enthaltendes, insbesondere Methacrylsäurereiches - und Methacrolein-haltiges Prozessgas 2 erhalten wird.
C) Trennung des Prozessgases 2 aus Verfahrensschritt B) durch Kondensation oder Quentschen, Extraktion und/oder Destillation in eine Phase 3a, enthaltend Methacrylsäure und eine flüssige Phase 3b, enthaltend Methacrolein.
D1) oxidative Veresterung des Methacroleins in der aus Verfahrensschritt C) erhaltenen Phase 3b in einem Reaktor 4 mit einem Alkohol in Gegenwart eines sauerstoffhaltigen Gases und eines heterogenen, Metalle und/oder Metalloxide umfassenden, edelmetallhaltigen Oxidationskatalysators, wobei eine Mischung, enthaltend ein Alkylmethacrylat, nicht umgesetzter Alkohol, Methacrylsäure und nicht umgesetztes Methacrolein, als flüssiger Prozessstrom 4 erhalten wird.
D2) eine sauer katalysierte Veresterung der Methacrylsäure der aus Verfahrensschritt C) erhaltenen, optional zusätzlich gereinigten Phase 3a mit einem Alkohol in einem Reaktor 3 an einem Kontakt III.
   und
D3) Reinigungsschritte zur Isolierung der in Phase 3a enthaltenen Methacrylsäure, die mindestens eine Destillation umfassen.

Weiterhin bevorzugt wird das Methacrolein-haltige Prozessgas 1, bevor es als aufgereinigtes Kondensat und nach Verdampfung als Teil des Prozessgas 1 in Verfahrensschritt B) eingesetzt wird, nach Schritt A) mittels mindestens einer Destillation und/oder Extraktion aufgereinigt.

In einer besonderen Variante der vorliegenden Erfindung handelt es sich bei dem sauerstoffhaltigen Gasstrom in Prozessschritt B) oder D1) um einen teilweise recyclierten Gasstrom. Es kann sich in dem Verfahren auch bei beiden genannten Gasströmen um einen teilweise recyclierten Gasstrom handeln.

Bevorzugt wird in zur oxidativen Veresterungsreaktion gemäß Schritt D1) ein heterogener Oxidationskatalysator eingesetzte. Besonders bevorzugt ist dieser Katalysator dadurch gekennzeichnet, dass er eines oder mehrere, bevorzugt ultrafein verteilte Metalle mit einer durchschnittlichen Teilchengröße von < 20 nm umfasst. Dies ist insbesondere der Fall, wenn der Katalysator Gold als eine aktive Komponente des Oxidationskatalysators enthält. Bei der Verwendung partikulärer Katalysatoren, die als eine aktive Komponente Palladium oder Platin enthalten, sind diese Komponenten nicht mehr zwingenderweise nanoskalig bzw. kleiner 20 mikron, sondern liegen auch in größeren Aggregaten vor. Üblicherweise weisen aber die Palladium- und Platinhaltigen Kontakte höhere Beladungen an Edelmetall auf als z.B. im Fall der Goldbasierten Kontakte. Es hat sich als besonders vorteilhaft erwiesen, wenn es sich bei diesen Metallen um Edelmetalle wie z.B. Palladium handelt. Besonders bevorzugt sind die Metalle ausgewählt aus der Gruppe bestehend aus Gold, Palladium, Ruthenium, Rhodium und Silber. In der Regel, aber nicht zwingend, wird nur eines dieser Metalle verwendet. Genauso bevorzugt ist es, die Reaktion in Schritt D1) bei einem Druck von 1 bis 100 bar in flüssiger Phase durchzuführen.

Genauso bevorzugt und insbesondere in Kombination mit dem zuvor genannten, weist der zur oxidativen Veresterungsreaktion gemäß Schritt D1) eingesetzte heterogene Oxidationskatalysator eines oder mehrere, bevorzugt ultrafein verteilte Metalle, insbesondere Edelmetalle auf einem oder mehreren Trägermaterialien, basierend auf Siliziumdioxid, Aluminiumoxid, Titandioxid, Magnesiumoxid, Bismuthoxid, Telluroxid, oder weiteren basischen Oxiden aus den Gruppen der Alkali und Erdalkalimetalle, wobei das entstehende Trägermaterial einen Durchmesser von 10 µm bis 10 mm aufweist, auf.

Besonders aktive Varianten dieser Katalysatoren, sind solche, die neben der Edelmetallkomponente eines oder mehrere weitere Elemente oder deren Oxide, ausgewählt aus der Gruppe von Mangan, Eisen, Cobalt, Nickel, Kupfer, Zink, Blei, den Lanthanoiden (Ordnungszahl 57 bis 71), Tellur, Antimon und Bismuth aufweist. Bekannte Katalysatoren basieren zum Beispiel auf dem oxidischen Trägern mit Palladium-Blei, Gold-Nickel, Gold-Cobalt oder Palladium mit einer Mischung aus Tellur, Antimon und Bismuth.

Sehr bevorzugt ist es, die Schritte A) bis D1), sowie D2) in einem kontinuierlichen Verfahren durchzuführen. Dies kann optional auch bezüglich Schritt D3) erfolgen, wobei dieser auch in einer Batch-Fahrweise in einem ansonsten kontinuierlichen Verfahren realisiert werden kann. Bezüglich D3) wäre es alternativ auch denkbar einen Teilstrom der Phase 3a abzuleiten und aus diesem Teilstrom, bevorzugt flexibel einstellbar, kontinuierlich oder in einem Batch gemäß D3) Methacrylsäure zu isolieren.

Das erfindungsgemäße Verfahren ist großtechnisch insbesondere relevant einsetzbar, wenn es sich bei dem Alkohol in Prozessschritt D1) um Methanol handelt. Bevorzugt wird bei dieser Ausführung die oxidative Veresterungsreaktion gemäß Schritt D1) mit einem molaren Verhältnis von Methanol zu Methacrolein in der stationären Reaktionsphase im Bereich von 1:1 bis 50:1 durchgeführt.

Unabhängig, bevorzugt aber in Kombination dazu wird die Umsetzung gemäß Schritt D1) bei einem Druck im Bereich von 2 bis 50 bar, einem pH-Wert im Bereich von 3 bis 10 und bei einer Temperatur im Bereich von 10 bis 200 °C in flüssiger Phase durchgeführt.

Die Alkohole in Prozessschritten D1) und D2) können unterschiedliche sein. Bei einer solche Ausführung erhält man entsprechend zwei verschiedene Methacrylsäureester, die getrennt voneinander isoliert und gereinigt werden können. Bevorzugt ist es jedoch, wenn es sich bei dem Alkohol in den Prozessschritten D1) und D2) jeweils um den gleichen Alkohol, besonders bevorzugt um Methanol handelt. Eine solche Ausführung hat insbesondere den Vorteil, dass die beiden Produktströme aus D1) und D2) zusammengeführt und gemeinsam aufgearbeitet werden können.

Besonders bevorzugt werden die Rohprodukte der Prozessschritte D1) und D2) direkt aus den Reaktoren 4 und 3 oder nach jeweils ein oder zwei optionalen separaten Reinigungsschritten zusammengeführt und gemeinsam gereinigt.

Insbesondere bei einem kontinuierlichen Betrieb des Verfahrens hat es sich als vorteilhaft erwiesen, wenn die jeweiligen organischen Phase aus Prozessschritten D1) und D2) getrennt in mindestens einem Destillationsschritt und/oder einem Extraktionsschritt gereinigt werden, bevor sie zusammengeführt werden. Aufgrund sehr unterschiedlicher Nebenprodukt-Charakteristika kann eine zunächst getrennte Aufarbeitung effizienter gestaltet werden. So kann es beispielsweise von Vorteil sein, dass Produkt der Stufe D1) erst niedrigsiedende Nebenprodukte und Edukte, insbesondere Methacrolein und Methanol abzutrennen und diese beispielsweise zurück in den Reaktor 4 zu leiten, was am Ende zu einer höheren Gesamtausbeute führt. Nebenprodukte bzw. Edukte aus dem Verfahrensschritt D2), insbesondere Methacrylsäure, können dagegen in Verfahrensschritt D1) im kontinuierlichen sehr störend sein, und beispielsweise den Katalysator auf Dauer schädigen.

Für den Reaktoraustrag der D2) dagegen wäre eine getrennte Aufarbeitung nur von geringerem Vorteil. Da hier jedoch mehr Nebenprodukte und Edukte vorliegen, benötigt man zur Reinigung normalerweise mehr Schritte als für das Produkt des Schrittes D1). Daher kann es hier von Vorteil sein, vor dem Zusammenführen der beiden Produktströme, nacheinander erst Hoch- und dann Niedrigsieder aus dem Rohprodukt des Schrittes D2) zu entfernen, bevor man beide Produktströme für eine weitere Aufarbeitung zusammenführt.

Zusammengefasst führt das erfindungsgemäße Verfahren zu diversen Vorteilen gegenüber Verfahren des Standes der Technik.

Gegenüber bekannten, von C4-Bausteinen ausgehenden Verfahren zur Herstellung von Alkylmethacrylaten, wie MMA, führt das erfindungsgemäße Verfahren zu einer überraschend hohen Gesamtausbeute. Dies ist insbesondere der Fall, da das nur teilweise Methacrolein aus Stufe B) in Stufe C) isoliert und in Stufe D1) sehr effizient umgesetzt wird. Wobei Stufe D1) grundsätzlich aufwendiger zu betreiben ist, als Stufen C) und D2), da für D1) in der Regel relativ teure Edelmetall-basierte Katalysatoren verwendet werden.

Dadurch ergibt sich auch ein insgesamt sehr hoher Gesamtumsatz der Edukte und somit eine relativ geringe Abfallmengen.

Überraschend wurde zudem gefunden, dass durch die Kombination zweier getrennter Routen zur Herstellung des MMA insgesamt nur geringe Konzentrationen an spezifischen Nebenprodukten im Endprodukt vorliegen. Selbst wenn die Gesamtmenge an Nebenprodukten vergleichbar sein sollte, so ist die Konzentration an einzelnen Substanzen durchaus relevant, z.B. bezüglich der Färbung des Endproduktes, bzw. aus diesem hergestellter Polymere, der Lagerstabilität oder einer Inhibitorwirkung bei einer Polymerisation

Weiterhin ist es mit dem erfindungsgemäßen Verfahren überraschend möglich, im kontinuierlichen und großtechnischen Betrieb sehr flexibel zwei unterschiedliche Alkylmethacrylate und zusätzlich sogar - über einen Teilstrom - Methacrylsäure gleichzeitig zu produzieren.

### Spezifische Aspekte des Verfahrens

Verfahrensgemäß ist die Erzeugung von Recycle-MALs und dessen Konditionierung zur Einstellung wichtiger, kritischer bzw. relevanter Konzentrationen von Methacrolein selbst und prozessspezifischen Nebenprodukten. Im Folgenden wird die Synthese von Recycle-MAL kurz nach gängigem Stand der Technik beschrieben.

In einem ersten Rohrbündelreaktor wird Isobuten bzw. tert-Butanol bei Temperaturen zwischen 320 bis über 400 °C bei leichtem Überdruck in Anwesenheit von Luftsauerstoff und Wasserdampf, sowie Recycle-Gas zu Methacrolein oxidiert. Der Umsatz liegt beim Tandemverfahren bei größer 98% bzw. beim "Separate C₄ Direct Oxidation" Verfahren tendenziell niedriger. Üblicherweise liegt die Verweilzeit im Reaktor mit modernen dotierten Bismuth-Molybdat-Kontakten bei 1 bis 4 Sekunden. Dies kann beispielsweise in der US 5,929 275 nachgelesen werden. Es werden GHSV-Werte zwischen 1000 und 2000 s⁻¹ erreicht. Die austretende Prozessgasphase wird mit einer kühleren Recycle-MAL Gasphase zusammen mit Luftsauerstoff und Wasserdampf vermischt. Somit ergibt sich das Feedgas der zweiten Stufe. Die zweite Oxidationsstufe wird wie die Erste bei einem moderaten Überdruck zwischen 0.1 und 2 bar und bei Temperaturen zwischen 260 und 360 °C betrieben. Hierzu kommen Heteropolysäurekontakte auf Basis von Molybdän und Phosphor sowie einigen weiteren Dopanten zum Einsatz (siehe dazu z.B. US2007/0010394). Die modifizierten Heteropolysäuren weisen nach wie vor eine große Abhängigkeit von Selektivität und Umsatz auf. Dies gilt insofern, dass bei höheren Umsätzen tendenziell signifikant schlechtere Selektivitäten erreicht werden. Aus diesem Grund wird der Umsatz und die damit verbundene Katalysatorbelastung zwischen 65 und 85% eingestellt. Aus dieser Tatsache ergibt sich für alle Verfahren und deren Modifikationen die Notwendigkeit nicht umgesetztes Methacrolein aus dem Prozessgas vom gewünschten Produkt Methacrylsäure abzutrennen und letztlich als sogenanntes Recycle-MAL vor den zweiten Oxidationsreaktor zurückzufahren.

Das von der Methacrylsäure getrennte Methacrolein-haltige Gemisch nach der zweiten Reaktionsstufe enthält je nach Katalysatorgüte und Parametern der Verfahrensdurchführung neben dem Methacrolein noch weitere Nebenprodukte wie unter anderem Aldehyden, die in einer oxidativen Veresterung (DOE) umgesetzt werden können. Das Methacrolein-haltige Gemisch wird im Folgenden als Recycle-MAL bezeichnet.

Als Stand der Technik kann für das Recycle-MAL folgendes Nebenproduktspektrum eingegrenzt werden:

| | |
|---|---|
| 0,5 - 4 Gew% | Acetaldehyd |
| 1 - 8 Gew% | Aceton |
| 1 - 5 Gew% | Acrolein |
| 0,05 - 0,4 Gew% | Butan-2,3-dion |
| 0,2 - 1,5 Gew% | MMA |
| 1 - 5 Gew% | Wasser |
| 1 - 5 Gew% | Methacrylsäure |
| 0,1 - 3 Gew% | Essigsäure |
| 70 - 95 Gew% | Methacrolein |

Kennzeichnend für das Recycle-MAL ist je nach Verfahren (Tandem- oder Zwischenisolierung von MAL) ein Methacroleingehalt größer 70 Gew% und enthaltend sowohl leichter siedenden Komponenten wie Aceton, Acrolein und Acetaldehyd, als auch höher siedende Komponenten wie MMA, Wasser und Methacrylsäure.

Als der oxidativen Veresterung der Stufe D1) zugängliche Aldehyde sind hier Acrolein sowie Acetaldehyd zu nennen. Weiterhin führen die Methacrylsäure und eventuelle weitere Säuren wie Essigsäure im Zulauf von Reaktor 4 zu einem höheren Bedarf nach Base um den gewünschten pH-Wert der oxidativen Veresterung einzustellen. Es ist also wünschenswert bei der Abtrennung von Methacrylsäure von Recycle-Methacrolein, die Methacrylsäurekonzentration im Recycle Methacrolein vor Verdampfung möglichst niedrig einzustellen.

Im erfindungsgemässen Verfahren ist neben der Umsetzung des Recycle-Methacroleins zu MMA in einer direkten oxidativen Veresterung zu MMA auch die Abtrennung von Recycle-Methacrolein von Methacrylsäure aus dem Prozessgas der zweiten Gasphasen Reaktion sehr bevorzugt. Das heiße Prozessgas 2 aus Schritt B tritt üblicherweise bei 250 bis 360°C aus dem Reaktor aus und muss zunächst abgekühlt werden. Üblicherweise wird zunächst über einen rekuperativen Gaskühler auf eine Temperatur zwischen 150-250°C abgekühlt. Rekuperative Gaskühler sind bevorzugt, weil mit diesen die Wärme zur Dampfgenerierung genutzt wird. Danach wird die jetzt in der Temperatur reduzierten Gasphase üblicherweise bei Temperaturen zwischen 50 bis 100 °C in eine umlaufende kondensierte Quenchphase geleitet. Diese Quenchphase kann der Sumpfteil einer Quenchkolonne sein, die über eine Pumpe umgewälzt und thermostatisiert wird. Am Kopf dieser Quenchkolonne geht der größte Teil des Methacroleins gasförmig zusammen mit dem Prozessgas über, während der Großteil der gebildeten Methacrylsäure im Sumpf kondensiert und gequencht wird. In einem nächsten Verfahrensschritt wird Methacrolein kondensiert bzw. zusammen mit Wasser absorbiert. In diesem Schritt fällt das Recycle Methacrolein, zusammen mit allen kondensierbaren Nebenkomponenten, wie z.B. Leichtsiedern, flüssig an. Trotzdem erreicht man eine effektive Abtrennung vom Prozessgas, das am Kopf dieser Kolonne entweicht. In einem letzten Schritt wird Methacrolein jetzt aus der Absorberphase desorbiert und man gelangt zum Reycle-Methacrolein, welches eine Reinheit von größer 70 Gew% aufweist. Exemplarisch wird so ein rohes Recycle-Methacrolein erzeugt, das jetzt der direkten oxidativen Veresterung zugeführt werden kann. Überraschend wurde gefunden, dass auch die Nebenprodukte im Recycle-Methacrolei, insbesondere reaktive Leichtsieder wie Acrolein und Acetaldehyd sowie andere Komponenten, ohne substanzielle Auswirkungen auf die Selektivität der Hauptreaktion oder auf die Katalysatorperformance allgemein so umgesetzt werden, dass die Nebenreaktionsprodukte effektiv vom gewünschten MMA abgetrennt werden können.

So kann Acrolein zu Methylacrylat, Acrylsäure, Acroleindimethylacetal, Methyl-3-methoxypropionat, Methyl-3-hydroxypropionat, Methyl-2-hydroxypropionat oder Hetero-Diels-Alder-Produkten zweier Acroleine, die als freie Säure oder Methylester vorliegen können, reagieren.

Acetaldehyd kann zu Essigsäure oder Methylacetat weiterreagieren.

Methacrolein kann neben den gewünschten Produkt MMA, beispielsweise auch zu in der Mischung mit MMA nicht gewünschter Methacrylsäure weiterreagieren. Weitere Folgeprodukte können daneben das Acetal di-Methoxyisobuten, Methyl-3-methoxyisobutyrat, Methyl-3-hydroxyisobutyrat, Methyl-2-hydroxyisobutyrat oder auch hier die entsprechenden Hetero-Diels-Alder-Produkte, die hier einfachhalber als di-MAL-Säure bze. Di-MAL-Ester bezeichnet werden, sein.

Zudem ist die Entfernung von Butan-2,3-dion (so genanntes Diacetyl) nach der DOE notwendig, weil Diacetyl in der Polymerisation von MMA zu PMMA zur Gelbfärbung der ansonsten transparenten PMMA Produkte führt. Diacetyl stammt aus den beiden Gasphasenoxidationsreaktionen und wird in die DOE mit dem Feedstock Recycle Methacrolein eingeschleppt. Schon in Spuren deutlich unterhalb 1 Gew% kann Diacetyl in späteren Polymeren zu Verfärbungen führen. Die DOE selbst bildet weitestgehend keine zusätzliches Diacetyl.

Die DOE des Recycle-MALs kann mit verschiedenen Alkoholen durchgeführt werden und liefert die entsprechenden Carbonsäureester aus den Aldehyden im Recyle-MAL sowie dem eingesetzten Alkohol. Als Alkohol wird bevorzugt Methanol eingesetzt. Alternativ kann auch ein di-, tri-oder tetra-funktioneller Alkohol eingesetzt werden. Die erhaltenen mehrfunktionalen Carbonsäureester sind als Vernetzer bekannt. Ein besonders bevorzugtes Beispiel für einen di-funktionellen Alkohol ist Ethylenglykol.

Die DOE kann dabei batchweise und kontinuierlich durchgeführt werden, wobei die kontinuierliche Durchführung besonders bevorzugt ist. Die Reaktionsführung kann dabei in verschiedenen, dem Fachmann bekannten, Reaktortypen durchgeführt werden. Als Beispiele, aber nicht limitierend, sind Rührkesselreaktoren, Blasensäulenreaktoren, Wirbelbettreaktoren, Rohrreaktoren, Rohrbündelreaktoren, Festbettreaktoren, Rieselbettreaktoren und alle Kombinationen daraus zu nennen. Ganz besonders bevorzugt wird der Katalysator während der DOE in einem gerührten Reaktor in Suspensionsform (als Slurry) angewendet.

Die folgenden Beispiele dokumentieren die Möglichkeit das Recycle-MAL in einer DOE umsetzen.

### Beispiele

### Beispiel 1 - Katalysatorträgerherstellung -Silika-Aluminiumoxid-Magnesiumoxid:

In einem 250 mL Becherglas werden 21,36 g Mg(NO₃)₂*6H₂O, 31,21 g Al(NO₃)₃*9H₂O zusammen vorgelegt und in 41,85 g VE Wasser unter Rühren mit einem Magnetrührer gelöst. Danach werden 1,57 g 60%ige HNO₃ unter Rühren zugegeben. 166,67 g Silicasol (Köstrosol 1530AS von der Firma Bad Köstritz, 30 Gew% SiO₂, Mittelgröße der Partikel: 15 nm) werden in einen 500 mL Dreihalskolben eingewogen und unter Rühren auf 15 °C gekühlt. 2,57 g 60%ige HNO₃ werden unter Rühren langsam zum Sol zugegeben. Bei 15 °C wird die Nitratlösung innerhalb von 45 min zum Sol unter Rühren zugegeben. Nach der Zugabe wird das Gemisch innerhalb von 30 min auf 50 °C erhitzt und weitere 24 h bei dieser Temperatur gerührt. Nach dieser Zeit wird das Gemisch bei 130 °C Ausgangstemperatur sprühgetrocknet. Das getrocknete Pulver (sphärisch, mittlere Partikelgroße 60 µm) wird in dünner Schicht im Naberofen innerhalb von 2h auf 300 °C erhitzt, 3 h bei 300 °C gehalten, innerhalb von 2 h auf 600 °C erhitzt und schließlich für 3 h bei 600 °C gehalten.

### Beispiel 2 - Katalysatorherstellung - AuCoO@Silika-Aluminiumoxid-Magnesiumoxid:

Eine Suspension von 10 g SiO₂-Al₂O₃-MgO des Trägers aus Beispiel 1 in 33,3 g VE-Wasser wird auf 90 °C aufgeheizt und 15 min bei dieser Temperatur gerührt. Zu dieser Suspension wird unter Rühren eine vorher auf 90 °C erhitzte Lösung von Co(NO₃)₂*6H₂O (569 mg, 1,95 mmol) in 8,3 g Wasser zugegeben. Nach der Zugabe wurde das Gemisch weitere 30 min bei 90 °C gerührt. Diese Suspension wird unter Rühren mit einer vorher auf 90 °C erhitzten Lösung von 0,52 mL einer einmolaren NaOH-Lösung versetzt. Direkt im Anschluss wird eine auf 90 °C erhitzte Lösung von HAuCl₄*3H₂O (205mg) in 4,3 g Wasser zugegeben. Nach der Zugabe wird die Suspension für weitere 30 Minuten gerührt, auf Raumtemperatur abgekühlt und filtriert. Der Filterkuchen wird mit VE-Wasser gewaschen bis die Leitfähigkeit unter 100 µS/cm gesunken ist. Das Material wird bei 105 °C für 10 Stunden getrocknet, die Agglomerate leicht zerstoßen und im Anschluss innerhalb von 1 Stunde auf 450 °C erhitzt, für 5 Stunden bei dieser Temperatur kalziniert und auf Raumtemperatur abgekühlt.

Für alle folgenden Beispiele war die Zusammensetzung des Recycle-MALs wie folgt:

| | |
|---|---|
| 2,0 Gew% | Acetaldehyd |
| 5,5 Gew% | Aceton |
| 3,5 Gew% | Acrolein |
| 0,2 Gew% | Diacetyl |
| 1,2 Gew% | MMA |
| 4,0 Gew% | Wasser |
| 2,9 Gew% | Methacrylsäure |
| 0,2 Gew% | Essigsäure |
| 80 Gew% | Methacrolein |

sonstige organische Bestandteile sind jeweils kleiner 0,1 Gew% und in Summe 2,5 Gew%

### Beispiel 3 - Batchtest:

Der in Beispiel 2 erhaltene Katalysator (384 mg), Recycle-MAL (1,20 g, 80 Gew% MAL) und Methanol (9,48 g) wurden in einem 140 mL Stahlautoklaven mit einem Magnetrührer suspendiert. Der pH-Wert des Recycle-MALs war 4,8 und wurde mit 100 ppm Tempol stabilisiert. Auf den Autoklaven wurden 30 bar Überdruck gepresst mit einer Gasmischung aus 7% O₂ in N₂. Das Explosionslimit der Mischung liegt 8 Vol% Sauerstoff. Der Autoklav wurde für 2 Stunden auf 60 °C erhitzt, abgekühlt, entgast und die Suspension filtriert. Das Filtrat wurde mittels GC analysiert. Der Umsatz an MAL war 17,9%, die Selektivität zu MMA 73,3% und die Raum-Zeit Ausbeute war 2,78 mol MMA / kg Katalysator pro Stunde.

Trotz nicht optimaler Parameterführung und keiner pH-Wert Regulierung während der Reaktion erkennt man die prinzipielle Eignung der DOE für Umsetzung von Recycle-MAL.

### Beispiel 4 - Batchtest:

Der in Beispiel 2 erhaltene Katalysator (384 mg), Recycle-MAL (1,20 g, 80 Gew% MAL) und Methanol (9,48 g) wurden in einem 140 mL Stahlautoklaven mit einem Magnetrührer suspendiert. Der pH-Wert des Recycle-MALs wurde mit 1% NaOH in MeOH zunächst auf 7,0 eingestellt und mit 100 ppm Tempol stabilisiert. Auf den Autoklaven wurden auf 30 bar Überdruck gepresst mit einer Gasmischung aus 7 Vol% O₂ in N₂. Das Explosionslimit der Mischung liegt bei 8 Vol% Sauerstoff. Der Autoklav wurde für 2 Stunden auf 60 °C erhitzt, abgekühlt, entgast und die Suspension filtriert. Das Filtrat wurde mittels GC analysiert. Der Umsatz an MAL war 28,9%, die Selektivität zu MMA 75,6% und die Raum-Zeit Ausbeute war 4,29 mol MMA / kg Katalysator pro Stunde.

### Beispiel 5 - Batchtest:

Der in Beispiel 2 erhaltene Katalysator (384 mg), Recycle-MAL (1,20 g, 80 Gew% MAL) und Methanol (9,48 g) wurden in einem 140 mL Stahlautoklaven mit einem Magnetrührer suspendiert. Der pH-Wert des Recycle-MALs wurde mit 1% NaOH in MeOH zunächst auf 7,0 eingestellt und mit 100 ppm Tempol stabilisiert. Auf den Autoklaven wurden auf 30 bar Überdruck gepresst mit einer Gasmischung aus 7 Vol% O₂ in N₂. Das Explosionslimit der Mischung liegt bei 8 Vol% Sauerstoff. Der Autoklav wurde für 2 Stunden auf 80 °C erhitzt, abgekühlt, entgast und die Suspension filtriert. Das Filtrat wurde mittels GC analysiert. Der Umsatz an MAL war 67,0%, die Selektivität zu MMA 89,5% und die Raum-Zeit Ausbeute war 11,1 mol MMA / kg Katalysator pro Stunde.

### Beispiel 6 - Batchtest:

Der in Beispiel 2 erhaltene Katalysator (384 mg), Recycle-MAL (1,20 g, 80 Gew% MAL) und Methanol (9,48 g) wurden in einem 140 mL Stahlautoklaven mit einem Magnetrührer suspendiert. Der pH-Wert des Recycle-MALs wurde mit 1% NaOH in MeOH zunächst auf 7,0 eingestellt und mit 100 ppm Tempol stabilisiert. Auf den Autoklaven wurden auf 30 bar Überdruck gepresst mit einer Gasmischung aus 7 Vol% O₂ in N₂. Das Explosionslimit der Mischung liegt bei 8 Vol% Sauerstoff. Der Autoklav wurde für 4 Stunden auf 60 °C erhitzt, abgekühlt, entgast und die Suspension filtriert. Das Filtrat wurde mittels GC analysiert. Der Umsatz an MAL war 47,3%, die Selektivität zu MMA 83,9% und die Raum-Zeit Ausbeute war 8,1 mol MMA / kg Katalysator pro Stunde.

### Beispiel 7 - Kontinuierliche Durchführung:

Recycle-MAL (80 Gew% MAL) und Methanol werden so gemischt, dass ein molares Verhältnis von 1 zu 4 (MAL zu MeOH) erhalten wird. Die Lösung wird unter Rühren und Kühlung auf pH = 7,0 eingestellt und mit 100 ppm Tempol stabilisiert. In einen Stahlautoklaven (400 mL) werden Katalysator aus Beispiel (20g, 7 Gew%) und Methanol gefüllt. Der Autoklav ist mit 2 kontinuierlichen Filtern, einem gaseinziehenden Rührer und einer Lufteinperlung ausgerüstet. Der Reaktor wird verschlossen, auf 4 bar mit Luft aufgepresst und auf 80 °C erwärmt. Die kontinuierliche Förderung von Recycle- MAL wurde so eingestellt, dass sich eine Belastung von 11 mol MAL / kg Katalysator pro Stunde ergibt. Durch Zugabe von 1 Gew% NaOH in Methanol wurde der pH-Wert auf 7,0 konstant gehalten. Die Reaktion wurde für 500 Stunden betrieben und alle 24 Stunden die kontinuierliche entnommen Produktproben per GC analysiert. Der Umsatz an MAL war 69%, die MMA Selektivität 93,5% und die Raum-Zeit-Ausbeute lag bei 7,1 mol MMA / kg Katalysator pro Stunde. Nach 500 Stunden konnte keine Katalysatordesaktivierung beobachtet werden und der Reaktor zeigte keine Verunreinigungen oder Anhaftungen von Polymer. Neben MMA wurden als Hauptnebenprodukte in einer Selektivität von 3,1 % Methacrylsäure und 2,4 % 3-Methoxyisobuttersäuremethylester in der Mischung gefunden. Daneben konnten Methylacrylat und Methylacetat als Nebenprodukte identifiziert werden. Aceton und Diacetyl, die mit dem Recycle-MAL schon im Zulauf vorhanden waren, wurden unter diesen Bedingungen, soweit messbar, nicht umgesetzt.

Die Beispiele, insbesondere Beispiel 7, zeigen, dass das erfindungsgemäße Verfahren gegenüber dem Stand der Technik für die Verwertung des Recycle-MALs deutliche Vorteile in Bezug auf Ausbeute und Kosten bringt und zugleich umweltschonender ist.

### Bezugszeichenliste

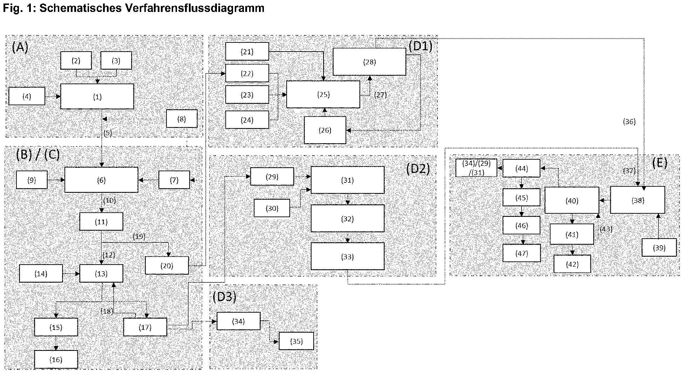

Fig.1 stellt schematisch Anlagenbestandteile zur Durchführung des erfindungsgemäßen Verfahrens dar. Im Rahmen der Erfindung können einzelne Ausführungen von dieser beispielhaften Darstellung durchaus abweichen.
**(A) Synthese und Isolierung des Methacroleins**
   ((1)-(5))
   (1) Reaktor 1 zur C4-Oxidation (Prozessschritt A)
   (2) Zustrom Wasserdampf
   (3) Sauerstoff- bzw. Luftzuleitung
   (4) Zulauf Isobuten und/oder tert-Butanol
   (5) Überführung des Prozessgases 1 in Reaktor 2
**(B) Oxidation des Methacrolein zu Methacylsäure und (C) Trennung MAS und MAL**
   ((6) - (20))
   (6) Reaktor 2 zur C4-Oxidation (Prozessschritt A)
   (7) Sauerstoff- bzw. Luftzuleitung
   (8) Optionale gemeinsame Kompression und Reinigung des Recycle-Gases für (5) bzw. (7)
   (9) Optionaler Zustrom Wasserdampf
   (10) Reaktoraustrag aus Reaktor 2 = Prozessgas 2
   (11) Quenschen und/oder Kondensieren des Prozessgases 2. Trennung des Prozessgases 2 in eine flüssige Methacrylsäure-haltige Phase 3 (hinzu (13)) und in eine gasförmige Methacrolein-haltige Phase 4 (hinzu (18))
   (12) flüssige Methacrylsäure-haltige Phase 3
   (13) Extraktion mit organischem Extraktionsmittel
   (14) Zuleitung organisches Extraktionsmittel (i.d.R. Heptan-Zulauf)
   (15) Wässrige Phase der Extraktion
   (16) Abwasserbehandlung
   (17) Organische Phase der Extraktion mit Roh-Methacrylsäure
   (18) Rückführung organisches Extraktionsmittel
   (19) gasförmige Methacrolein-haltige Phase 4
   (20) Aufreinigung Methacrolein mittels Absorption / Desorption
**(D1) Oxidative Veresterung des Methacroleins zu einem Alkylmethacrylat und Recyclierung des Methacroleins**
   ((21) - (27))
   (21) Zuleitung Alkohol (i.d.R. Methanol-Zulauf)
   (22) Bereitstellung Methacrolein, optional mit weiterer Destillation zur Entfernung von Niedrigsiedern
   (23) Sauerstoff- bzw. Luftzuleitung
   (24) Zulauf Base
   (25) Reaktor 4 zur oxidativen Veresterung des Methacroleins
   (26) Mischphase aus Methacrolein und Alkohol zur Zurückführung in Reaktor 4
   (27) Reaktoraustrag Reaktor 4 (23)
   (28) Destillationskolonne zur Trennung von Methacrolein und teilweise Alkohol von Roh-Alkymethacrylat
**(D2) Veresterung von Methacrylsäure zu einem Alkylmethacrylat**
   ((29)-(33))
   (29) Optionale weitere Aufreinigung des Phase 3 zur Entfernung von Leichtsiedern
   (30) Zuleitung Alkohol (i.d.R. Methanol-Zulauf)
   (31) Reaktor 3 zur Veresterung von Methacrylsäure zu einem Alkylmethacrylat
   (32) Optionale Destillation des Reaktoraustrags aus Reaktor 3 (24) zur Entfernung von Hochsiedern
   (33) Optionale Destillation des Reaktoraustrags aus Reaktor 3 (24) zur Entfernung von Niedrigsiedern
**(D3) Isolierung von Methacrylsäure**
   ((34)-(35))
   (34) Destillation der Roh-Methacrylsäure
   (35) Optionale weitere Aufarbeitung der Methacrylsäure
**(E) Beispielhafte Aufarbeitung des rohen Alkylmethacrylats (z.B. Roh-MMA)**
   ((36) - (47))
   (36) Zulauf Roh-Alkylmethacrylat aus Verfahrensschritt D1
   (37) Zulauf Roh-Alkylmethacrylat aus Verfahrensschritt D2
   (38) Phasentrenner mit optional vorgeschaltetem Mischer
   (39) Zulauf Säure und Wasser, optional getrennt
   (40) Extraktion
   (41) Destillation zur Rückgewinnung von Alkohol (und Methacrolein) zur optionalen Recyclierung
   (42) Sumpf zur Entsorgung oder weiteren Aufarbeitung
   (43) Optionale Recyclierung der Methacrylsäure-haltigen wässrigen Seitenstromfraktion aus (41) in (25)
   (44) Destillationskolonne zur Abtrennung von Schwersiedern (Methacrylsäurehaltiger Strom) zur optionalen Weiterleitung in (32), (27) oder (29))
   (45) Destillationskolonne zur Abtrennung von Leichtsiedern
   (46) Destillationskolonne zur Endreinigung des Alkylmethacrylats
   (47) Alkylmethacrylat-Produktstrom

Zu den Zeichnungen sei angemerkt, dass weitere dem Fachmann bekannte Komponenten zusätzlich in der zur Durchführung des erfindungsgemäßen Verfahrens enthalten sein können. So weist beispielsweise in der Regel jede der aufgeführten Kolonnen einen Kondensator auf.

Auch sei angemerkt, dass in den Zeichnungen nicht jede bevorzugte Ausführungsform berücksichtigt ist.

Die Position der Zuleitungen gibt nicht deren reale Lage an, sondern weist nur darauf hin in welchen Apparat die Zuleitung geführt wird.

## Patentansprüche

1. Verfahren zur Herstellung von Alkylmethacrylat und optional Methacrylsäure, bei dem in einer ersten Reaktionsstufe in einem Reaktor 1 Methacrolein durch eine Partialoxidation von Isobuten und/oder Tertbutylalkohol in der Gasphase hergestellt wird und dieses in einer zweiten Reaktionsstufe in einem Reaktor 2 durch Partialoxidation in der Gasphase zu Methacrylsäure umgesetzt wird, **dadurch gekennzeichnet, dass**
das in der zweiten Reaktionsstufe im Reaktor 2 nicht umgesetzte Methacrolein von der entstehenden Methacrylsäure abgetrennt wird und in einem weiteren Oxidationsschritt in einem Reaktor 4 in der Flüssigphase in Anwesenheit von einem Alkohol oxidativ verestert wird und
die in Reaktor 2 entstandene rohe Methacrylsäure destillativ und/oder extraktiv aufgereinigt wird und optional in einem weiteren Schritt mit einem Alkohol in einem Reaktor 3 sauer katalysiert zum Alkylmethacrylat umgesetzt wird.

2. Verfahren zur Herstellung von Alkylmethacrylat und optional Methacrylsäure, umfassend die Schritte:
A) Herstellung von Methacrolein aus Isobuten und/oder tert-Butanol in Gegenwart von Wasserdampf und einem sauerstoff-haltigen Gas in einem Reaktor 1, wobei die Reaktion in der Gasphase an einem heterogenen Kontakt I erfolgt, und wobei ein Methacrolein-haltiges Prozessgas 1 erhalten wird,
B) Überführung des Prozessgases 1, enthaltend Methacrolein und Wasserdampf, aus Reaktor 1 unter Zuführung eines sauerstoffhaltigen Gases und optional weiteren Wasserdampfes in einen Reaktor 2, wobei eine Reaktion an einem heterogenen Kontakt II erfolgt, und ein Methacrylsäure- und Methacrolein-haltiges Prozessgas 2 erhalten wird,
C) Trennung des Prozessgases 2 aus Schritt B) durch Kondensation oder Quentschen, Extraktion und/oder Destillation in eine Phase 3a, enthaltend Methacrylsäure und eine Phase 3b, enthaltend Methacrolein,
D1) oxidative Veresterung des Methacroleins in der aus Schritt C) erhaltenen Phase 3b in einem Reaktor 4 mit einem Alkohol in Gegenwart eines sauerstoffhaltigen Gases und eines heterogenen, Metalle und/oder Metalloxide umfassenden, edelmetallhaltigen Oxidationskatalysators, wobei eine Mischung, enthaltend ein Alkylmethacrylat, nicht umgesetzter Alkohol, Methacrylsäure und nicht umgesetztes Methacrolein, als flüssiger Prozessstrom 4 erhalten wird,
D2) eine sauer katalysierte Veresterung der Methacrylsäure der aus Schritt C) erhaltenen, optional zusätzlich gereinigten Phase 3a mit einem Alkohol in einem Reaktor 3 an einem Kontakt III und
D3) Reinigungsschritte zur Isolierung der in Phase 3a enthaltenen Methacrylsäure, die mindestens eine Destillation umfassen.

3. Verfahren gemäß Anspruch 2, **dadurch gekennzeichnet, dass** das Methacrolein-haltige Prozessgas 1 nach Schritt A) mittels mindestens einer Destillation und/oder Extraktion aufgereinigt wird, bevor es als aufgereinigtes Kondensat und nach Verdampfung als Teil des Prozessgas 1 in Verfahrensschritt B) eingesetzt wird.

4. Verfahren gemäß Anspruch 2 oder 3, **dadurch gekennzeichnet, dass** es sich bei dem sauerstoffhaltigen Gasstrom in Prozessschritt B) und/oder D1) um einen teilweise recyclierten Gasstrom handelt.

5. Verfahren gemäß mindestens einem der Ansprüche 1 bis 4, **dadurch gekennzeichnet, dass** der zur oxidativen Veresterungsreaktion gemäß Schritt D1) eingesetzte heterogene Oxidationskatalysator eines oder mehrere ultrafein verteilte Metalle mit einer durchschnittlichen Teilchengröße von < 20 nm umfasst, welches ausgewählt ist aus der Gruppe bestehend aus Gold, Palladium, Ruthenium, Rhodium und Silber, und dass die Reaktion in Schritt D1) bei einem Druck von 1 bis 100 bar in flüssiger Phase durchgeführt wird.

6. Verfahren gemäß mindestens einem der Ansprüche 1 bis 5, **dadurch gekennzeichnet, dass** der zur oxidativen Veresterungsreaktion gemäß Schritt D1) eingesetzte heterogene Oxidationskatalysator eine oder mehrere Edelmetalle auf einem oder mehreren Trägermaterialien, basierend auf Siliziumdioxid, Aluminiumoxid, Titandioxid, Magnesiumoxid, Bismuthoxid, Telluroxid, oder weiteren basischen Oxiden aus den Gruppen der Alkali und Erdalkalimetalle, wobei das entstehende Trägermaterial einen Durchmesser von 10 µm bis 10 mm aufweist.

7. Verfahren gemäß Anspruch 6, **dadurch gekennzeichnet, dass** neben der Edelmetallkomponente eines oder mehrere weitere Elemente oder deren Oxide, ausgewählt aus der Gruppe von Mangan, Eisen, Cobalt, Nickel, Kupfer, Zink, Blei, den Lanthanoiden, Tellur, Antimon und Bismuth aufweist.

8. Verfahren gemäß mindestens einem der Ansprüche 2 bis 8, **dadurch gekennzeichnet, dass** die Schritte A) bis D1), sowie D2) und optional D3) in einem kontinuierlichen Verfahren durchgeführt werden.

9. Verfahren gemäß mindestens einem der Ansprüche 2 bis 8, **dadurch gekennzeichnet, dass** es sich bei dem Alkohol in Prozessschritt D1) um Methanol handelt, und dass die oxidative Veresterungsreaktion gemäß Schritt D1) mit einem molaren Verhältnis von Methanol zu Methacrolein in der stationären Reaktionsphase im Bereich von 1:1 bis 50:1 erfolgt.

10. Verfahren gemäß mindestens einem der Ansprüche 2 bis 8, **dadurch gekennzeichnet, dass** die Umsetzung gemäß Schritt D1 bei einem Druck im Bereich von 2 bis 50 bar, einem pH-Wert im Bereich von 3 bis 10 und bei einer Temperatur im Bereich von 10 bis 200 °C in flüssiger Phase durchgeführt wird.

11. Verfahren gemäß mindestens einem der Ansprüche 2 bis 10, **dadurch gekennzeichnet, dass** es sich bei dem Alkohol in den Prozessschritten D1) und D2) jeweils um Methanol handelt.

12. Verfahren gemäß mindestens einem der Ansprüche 2 bis 11, **dadurch gekennzeichnet, dass** die Rohprodukte der Prozessschritte D1) und D2) direkt aus den Reaktoren 4 und 3 oder nach jeweils ein oder zwei optionalen separaten Reinigungsschritten zusammengeführt und gemeinsam gereinigt werden.

13. Verfahren gemäß mindestens einem der Ansprüche 2 bis 12, **dadurch gekennzeichnet, dass** die jeweiligen organischen Phasen aus Prozessschritten D1) und D2) getrennt in mindestens einem Destillationsschritt und/oder einem Extraktionsschritt gereinigt werden, bevor sie zusammengeführt werden.
